(19) Europäisches Patentamt
European Patent Office
Office européen des brevets

(11) **EP 1 197 236 B1**

(12) **EUROPÄISCHE PATENTSCHRIFT**

(45) Veröffentlichungstag und Bekanntmachung des Hinweises auf die Patenterteilung:
**23.01.2008 Patentblatt 2008/04**

(51) Int Cl.:
***A61M 1/28*** *(2006.01)*

(21) Anmeldenummer: **01123924.1**

(22) Anmeldetag: **06.10.2001**

(54) **Verfahren zur Bestimmung des Intraperitonealvolumens und Vorrichtung zur Peritonealdialyse**

Method for evaluating the intraperitoneal volume and apparatus for peritoneal dialysis

Méthode pour évaluer le volume intrapéritonéal et appareil de dialyse péritonéale

(84) Benannte Vertragsstaaten:
**DE ES FR GB IT**

(30) Priorität: **10.10.2000 DE 10049900**

(43) Veröffentlichungstag der Anmeldung:
**17.04.2002 Patentblatt 2002/16**

(73) Patentinhaber: **Fresenius Medical Care Deutschland GmbH**
**61352 Bad Homburg v.d.H. (DE)**

(72) Erfinder: **Noack, Joachim, Dr.**
**97616 Bad Neustadt (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**EP-B- 0 149 001      US-A- 5 643 201**
**US-A- 5 670 057**

## Beschreibung

Die Erfindung betrifft ein Verfahren zur Bestimmung des Intraperitonealvolumens während einer Peritonealdialyse und eine Vorrichtung zur Peritonealdialyse mit einer Einrichtung zur Bestimmung des Intraperitonealvolumens.

Bei der kontinuierlichen ambulanten Peritonealdialyse (CAPD) wird über einen permanent implantierten Peritonealkatheter Dialysat in den Peritonealraum, d. h. die Bauchhöhle des Patienten instilliert. Das Dialysat verbleibt mehrere Stunden in der Bauchhöhle. Nach Ablauf dieses Zyklus wird das Dialysat wieder abgeführt. Durch den Austausch von Flüssigkeit und gelösten Stoffen zwischen dem Blut in den peritonealen Kappilaren und dem Dialysat kann eine ausreichende Elimination harnpflichtiger Substanzen erfolgen.

Die bekannten Geräte für die sogenannte Continous Flow Peritoneal Dialysis (CFPD) verfügen über einen Dialysator, der durch eine Membran in eine erste und zweite Kammer geteilt ist. Die erste Kammer ist über einen ersten sterilen Kreislauf mit dem Peritonealkatheter verbunden, während die zweite Kammer über einen Kreislauf mit einer Vorrichtung zur Bereitstellung von Dialysierflüssigkeit verbunden ist. Die Kreisläufe sind durch die Membran des Dialysators voneinander getrennt. Während der Peritonealdialyse (CFPD) wird Peritoneallösung aus dem Peritonealraum an der einen Seite der Membran vorbeigeführt, während Dialysierflüssigkeit an der anderen Seite der Membran vorbeigeführt wird. An der Membran kommt es zu einem Stoffaustausch zwischen der Peritoneallösung und der kontinuierlich bereitgestellten, frischen Dialysierflüssigkeit. Dabei werden die membrangängigen Toxine aus der Peritoneallösung entfernt und umgekehrt fehlende, durch den Patienten resorbierte Substanzen, wie z. B. Glukose, wieder ausgeglichen.

Durch den Stoffaustausch werden der Konzentrationsgradient der Toxine und der osmotische Gradient der Lösung im Peritonealraum gegenüber der Umgebung aufrechterhalten, so daß eine stetige Sekretion von Flüssigkeit über das Peritoneum in den Peritonealraum stattfindet, was zu einer praktisch unbegrenzten Flüssigkeitszunahme im Peritonealraum führen kann. Diese Flüssigkeitszunahme wird nachfolgend auch als Patientenultrafiltration bezeichnet.

Unumgänglich zum Schutz des Patienten vor einer "Überfüllung" ist daher die Bestimmung des Intraperitonealvolumens, d. h. des Flüssigkeitsvolumens im Peritonealraum. Ist das Intraperitonealvolumen bekannt, kann die überschüssige Flüssigkeit dem ersten Kreislauf über die Membran des Dialysators wieder entzogen werden. Hierzu verfügen die bekannten CFPD-Geräte über eine Ultrafiltrationseinrichtung.

Das Intraperitonealvolumen kann empirisch oder durch einen Patientenauslauf ermittelt werden. Die EP-A-0 149 001 beschreibt ein Peritonealdialysegerät, bei dem die Ultrafiltrationsrate in Abhängigkeit von dem Intraperitonealvolumen gesteuert wird, so daß eine "Überfüllung" des Patienten ausgeschlossen ist. Die Ultrafiltrationskontrolle beruht auf einer Verdünnungsmessung. Das Peritonealdialysegerät umfaßt einen geschlossenen Kreislauf, in dem Dialysierflüssigkeit zirkuliert, der von außen eine Substanz zugesetzt wird, deren Sekretions- und Resorptionsrate während der gesamten Behandlungsdauer vernachlässigbar ist. Mit zunehmendem Volumen nimmt die Konzentration dieser Substanz in der Peritonellösung kontinuierlich ab. Während der Behandlung wird die Konzentration gemessen und mit der Anfangskonzentration zu Beginn der Behandlung verglichen. Wenn eine Differenz zwischen der gemessenen Konzentration und der Anfangskonzentration festgestellt wird, entzieht die Ultrafiltrationseinrichtung dem Peritonealraum so viel Flüssigkeit, bis sich in der Dialysierflüssigkeit wieder die Anfangskonzentration eingestellt hat.

Nachteilig ist, daß eine körperfremde Substanz der Dialyseflüssigkeit zugesetzt werden muß. Daher sind Unverträglichkeiten nicht auszuschließen. Nachteilig ist auch, daß eine Sekretion- oder Resorption der Substanz zu einer fehlerhaften Regelung der Ultrafiltrationsrate führt.

Der Erfindung liegt die Aufgabe zugrunde, ein Verfahren anzugeben, das eine einfache Bestimmung des Intraperitonealvolumens ohne die Gefahr von Unverträglichkeiten erlaubt. Eine weitere Aufgabe der Erfindung ist, eine Peritonealdialysevorrichtung zu schaffen, die eine einfache Bestimmung des Intraperitonealvolumens ohne Risiken für den Patienten ermöglicht.

Die Lösung dieser Aufgabe erfolgt erfindungsgemäß mit den im Patentanspruch 1 bzw. 6 angegebenen Merkmalen.

Die Bestimmung des Intraperitonealvolumens beruht auf der Konzentrationsmessung einer körpereigenen Substanz, die während der Peritonealdialyse über das Peritoneum in den Peritonealraum übertritt. Die Vorteile liegen darin, daß körperfremde Stoffe nicht zugesetzt werden müssen. Die Bestimmung des Intraperitonealvolumens ist daher weniger aufwendig und kostengünstiger. Ferner können Unverträglichkeiten ausgeschlossen werden.

Während der Peritonealdialyse strömt Peritoneallösung in einem ersten Kreislauf entlang der einen Seite einer Membran, während Dialysierflüssigkeit in einem zweiten Kreislauf an der anderen Seite der Membran entlangströmt. Unter einem Kreislauf wird nicht zwingend ein geschlossenes System, sondern auch ein System verstanden, bei dem Flüssigkeit aus einer Quelle zuströmt und in einen Ablauf abgeführt wird. Beide Kreisläufe können jeweils nochmals mehrere Teilkreisläufe umfassen.

Die Membran zwischen dem ersten und dem zweiten Kreislauf ist vorzugsweise für die körpereigene Substanz nicht durchgängig. Sollte die Membran dennoch durchgängig sein, ist die Verringerung der Konzentration der Substanz aufgrund des Durchtritts derselben durch die Membran bei der Auswertung der Meßergebnisse entsprechend zu korrigieren.

Eine geeignete körpereigene Substanz für die Konzentrationsmessung ist vorzugsweise Albumin. Aufgrund des hohen Molekulargewichts von über 60 kDalton wird Albumin nur sehr langsam über das Peritoneum transportiert, so daß sich die Albuminkonzentration durch Sekretions- oder Resorptionsprozesse im Patienten nur sehr langsam verändert. Außerdem sind in der Hämodialyse übliche Filtermembranen für diese Substanz nicht durchgängig.

Die Messung der Konzentration der körpereigenen Substanz in der Peritoneallösung kann grundsätzlich über die gesamte Behandlungsdauer erfolgen. Sie setzt aber voraus, daß die Sekretion- oder Resorption der Substanz zu vernachlässigen ist. Vorteilhafterweise werden zur Konzentrationsbestimmung zwei Messungen zu zwei unmittelbar aufeinanderfolgenden Zeitpunkten durchgeführt, wobei zwischen den Messungen ein vorgegebenes Volumen an Peritoneallösung dem ersten Kreislauf entzogen wird. Anstelle eines Flüssigkeitsentzuges kann auch eine kontrollierte Flüssigkeitszufuhr in den Patientenkreislauf erfolgen, so daß nicht nur die Aufkonzentration von Albumin als körpereigene Substanz gemessen werden kann, sondern auch eine Verdünnung. Das Intraperitonealvolumen wird dann aus den zu den beiden aufeinanderfolgenden Zeitpunkten gemessenen Konzentrationen und dem entzogenen Volumen berechnet. Dies hat den Vorteil, daß in diesem Zeitraum die Sekretion oder Resorption der körpereigenen Substanz zu vernachlässigen ist. Grundsätzlich kann daher die Verdünnungsmessung auch mit einer körperfremden Substanz erfolgen, deren Sekretion oder Resorption über die gesamte Behandlungsdauer nicht zu vernachlässigen wäre.

Aus der Änderung des Intraperitonealvolumens wird vorteilhafterweise die Ultrafiltrationsrate des Patienten bestimmt. Mit dieser Ultrafiltrationsrate wird dem ersten Kreislauf dann Flüssigkeit entzogen, um eine "Überfüllung" des Patienten zu vermeiden. Um die Ultrafiltrationsrate fortlaufend korrigieren zu können, werden vorzugsweise mehrere dieser Messungen über die gesamte Behandlungsdauer vorgenommen.

Die Einrichtung zur Bestimmung des Peritonealvolumens verfügt über eine Meßeinheit zur Bestimmung der Konzentration der körpereigenen Substanz sowie eine Rechen- und Auswerteinheit, die aus einer Konzentrationsänderung der körpereigenen Substanz das Intraperitonealvolumen bestimmt. Als Sensoren können beispielsweise Absorptions- oder Transmissions-Detektoren eingesetzt werden.

Im folgenden werden zwei Ausführungsbeispiele unter Bezugnahme auf die Zeichnungen näher erläutert.

Es zeigen:

Figur 1            ein erstes Ausführungsbeispiel einer Peritonealdialysevorrichtung mit einer Einrichtung zur Bestimmung des Peritonealvolumens in vereinfachter Darstellung,

Figuren 2a bis 2c    die Albuminkonzentration, das Intraperitonealvolumen und die Ultrafiltrationsrate während der Dialysebehandlung, und

Figur 3            ein zweites Ausführungsbeispiel der Peritonealdialysevorrichtung mit einer Einrichtung zur Bestimmung des Peritonealvolumens.

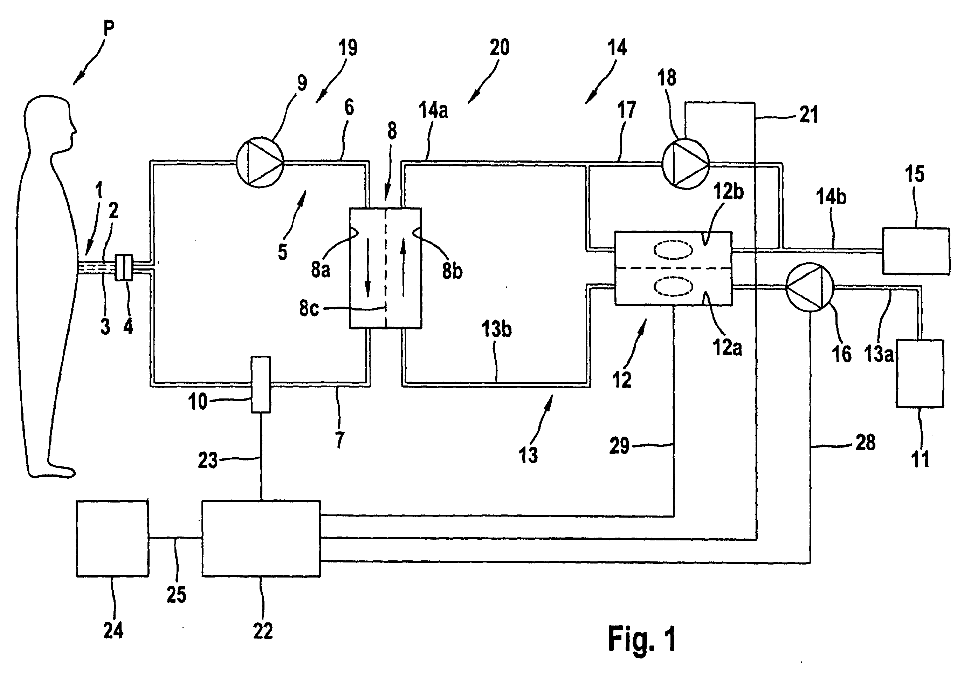

Figur 1 zeigt die wesentlichen Komponenten der Vorrichtung zur Peritonealdialyse in stark vereinfachter schematischer Darstellung. Die Peritonealdialysevorrichtung umfaßt einen permanent implantierbaren Peritonealkatheter 1, der ein erstes und zweites Lumen 2, 3 zum Zuführen einer Peritoneallösung in den Peritonealraum bzw. Abführen der Lösung aus dem Peritonealraum des Patienten aufweist. Der Peritonealkatheter 1 ist mittels eines Konnektors 4 mit einem Überleitungsschlauchsystem 5 verbunden, das eine Zulaufleitung 6 und eine Ablaufleitung 7 aufweist. Das eine Ende der Zulaufleitung 6 ist mit dem ersten Lumen 2 des Peritonealkatheters verbunden, während das andere Ende der Zulaufleitung mit dem Einlaß einer ersten Kammer 8a eines durch eine semipermeable Membran 8c in die erste Kammer und eine zweite Kammer 8b unterteilten Dialysators 8 verbunden ist. Der Auslaß der ersten Kammer 8a des Dialysators 8 ist an dem einen Ende der Ablaufleitung 7 angeschlossen, während das andere Ende der Ablaufleitung mit dem zweiten Lumen 3 des Peritonealkatheters 1 verbunden ist.

Zum Fördern der Peritoneallösung aus dem Peritonealraum des Patienten P befindet sich in der Zulaufleitung 6 eine okkludierende Schlauchpumpe 9. Eine Meßeinheit 10 mißt in der Peritoneallösung eine körpereigene Substanz, die während der Peritonealdialyse über das Peritoneum in den Peritonealraum übertritt, aber für die die Membran 8c des Dialysators nicht durchgängig ist. Die Peritoneallösung wird mittels der Pumpe 9 über die Zulaufleitung 6 aus dem Peritonealraum der ersten Kammer 8a des Dialysators 8 zugeführt und über die Ablaufleitung 7 und die Meßeinheit 10 wieder in den Peritonealraum zurückgeführt. Während in dem ersten Kreislauf 19 entlang der einen Seite der Membran 8c des Dialysators 8 Peritoneallösung strömt, fließt in dem zweiten Kreislauf 20 entlang der anderen Seite der Membran frische Dialysierflüssigkeit.

Die frische Dialysierflüssigkeit wird in einer Dialysierflüssigkeitsquelle 11 bereitgestellt. Zur Bilanzierung von frischer und verbrauchter Dialysierflüssigkeit ist eine nur schematisch dargestellte Bilanziereinrichtung 12 vorgesehen, die vorteilhafterweise zwei Bilanzkammern 12a, 12b enthält, die jeweils durch eine Membran in zwei Bilanzkammerhälften unterteilt sind. Eine derartige Bilanziereinrichtung ist beispielsweise aus der DE-A-28 38 414 bekannt.

Ein erster Abschnitt 13a einer Zuführleitung 13 verbindet die Dialysierflüssigkeitsquelle 11 mit der Bilanziereinrichtung 12, während der zweite Abschnitt 13b der Zuführleitung die Bilanziereinrichtung mit der zweiten Kammer 8b des Dialysators 8 verbindet. Der erste Abschnitt 14a einer Abführleitung 14 verbindet die zweite Kammer 8b des Dialysators 8 mit der Bilanziereinrichtung 12, während der zweite Abschnitt 14b der Abführleitung 14 die Bilanziereinrichtung mit einem Ablauf 15 verbindet. Von dem ersten Abschnitt 14a der Zuführleitung zweigt eine Ultrafiltrationsleitung 17 ab, die zu dem zweiten Abschnitt 14b der Zuführleitung führt. In der Ultrafiltrationsleitung 17 befindet sich eine Ultrafiltrationspumpe 18, mit der über die Membran 8c des Dialysators 8 dem ersten Kreislauf 19 Flüssigkeit entzogen werden kann. Eine Pumpe 16 in dem ersten Abschnitt 13a der Zuführleitung 13 fördert frische Dialysierflüssigkeit zur Bilanziereinrichtung 12.

Die Pumpe 16 ist über eine Steuerleitung 28 und die Ultrafiltrationspumpe 18 ist über eine Steuerleitung 21 mit einer Steuereinheit 22 verbunden, die wiederum über eine Datenleitung 23 mit der Meßeinheit 10 verbunden ist. Eine Rechen- und Auswerteinheit 24 ist über eine Datenleitung 25 mit der Steuereinheit verbunden. Die Steuereinrichtung 22 ist über eine Steuerleitung 29 auch mit der Bilanzierungseinrichtung 12 verbunden, so daß diese so gesteuert werden kann, daß dem ersten Kreislauf 5 eine definierte Flüssigkeitsmenge über den Dialysator 8 zugeführt wird.

Die Steuer-, Rechen- und Auswerteinheit sowie die Meßeinheit sind Bestandteil der Einrichtung zur Bestimmung des Intraperitonealvolumens. Rechen- und Auswerteinheit sowie Steuereinheit können ein Mikrocomputer sein, der in den bekannten Dialysevorrichtungen im allgemeinen ohnehin vorhanden ist. Zur Bestimmung des Intraperitonealvolumens arbeitet die Einrichtung wie folgt:

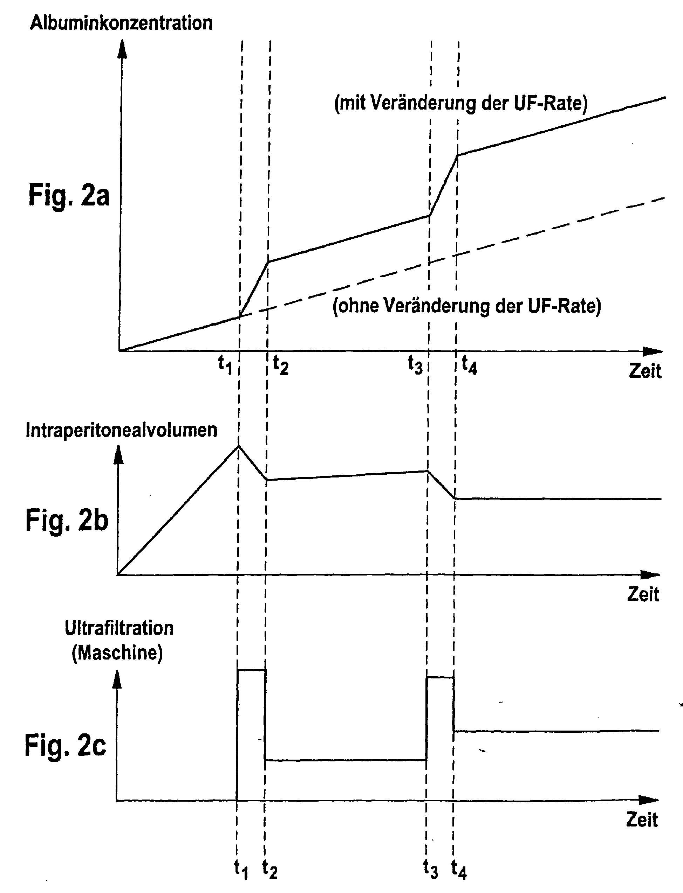

Die Konzentration von Albumin im Blut des Patienten liegt bei 40 g/l. Während der Dialyse kommt es zu einem konvektiven und diffusiven Transport von Albumin als körpereigene Substanz in die Peritoneallösung, so daß die Albuminkonzentration der Peritoneallösung kontinuierlich ansteigt. Aufgrund der großen Molekülgröße des Albumins von etwa 60 kDalton ist der Transport von Albumin aus dem Blut in die Peritoneallösung sehr langsam und stark vom konvektiven Transport bestimmt. Der zeitliche Verlauf der Albuminkonzentration während der Peritonealdialyse bei konstanter Glukosekonzentration der Peritoneallösung zeigt die gestrichelte Kurve in Figur 2a.

Die Steuereinheit 22 gibt nun den folgenden Meßablauf vor. Zunächst gibt die Steuereinheit 22 eine Ultrafiltrationsrate von 0 vor, d. h. die Ultrafiltrationspumpe 18 steht still (Figur 2c). Die Albuminkonzentration nimmt nun in der Peritoneallösung langsam zu (Figur 2a). Gleichzeitig nimmt auch das Intraperitonealvolumen zu (Figur 2b). Zum Zeitpunkt $t_1$ mißt die Meßeinheit 10 die Albuminkonzentration $c_0$ in der Peritoneallösung. Daraufhin wird die Ultrafiltrationsrate kurzzeitig erhöht, um den ersten Kreislauf 19 eine vorgegebene Menge an Flüssigkeit zu entziehen (Fig. 2c). In Versuchen hat sich gezeigt, daß der Entzug einer Flüssigkeitsmenge von 200 bis 800 ml für eine hinreichend genaue Bestimmung des Intraperitonealvolumens ausreichend ist. Zum Zeitpunkt $t_2$ mißt die Meßeinheit 10 wieder die Albuminkonzentration $c_1$.

Die Rechen- und Auswerteinheit 24 berechnet aus dem entzogenen Volumen $\Delta V$ und der Albuminkonzentration $c_0$ und $c_1$ das Intraperitonealvolumen V nach der folgenden Gleichung

$$V = \frac{\Delta V}{1 - c_0 / c_1}$$

Gleichzeitig berechnet die Rechen- und Auswerteinheit aus den Albuminkonzentrationen $c_0$, $c_1$ die Patientenultrafiltrationsrate V $(t_1)/t_1$. Im weiteren Verlauf der Behandlung gibt die Steuereinheit 22 dann eine Ultrafiltrationsrate vor, die der Patientenultrafiltrationsrate entspricht. Zu einem späteren Zeitpunkt $t_3$ wird die Messung des Intraperitonealvolumens nach dem oben beschriebenen Verfahren wiederholt und die Ultrafiltrationsrate der Maschine wird an die ggf. veränderte Patientenultrafiltrationsrate angepaßt. Entscheidend ist, daß der Anstieg der Albuminkonzentration zwischen den beiden Zeitpunkten $t_1$, $t_2$ bzw. $t_3$, $t_4$ zu vernachlässigen ist, so daß die Sekretion und Resorption von Albumin auf das Meßergebnis keinen Einfluß hat.

Sollte die Konzentration des zu messenden körpereigenen Stoffes während der Meßdauer nicht vernachlässigbar sein, kann das Meßergebnis durch die Konzentrationsmessungen zu weiteren Zeitpunkten $t_1$, $t_2$ z. B. linear korrigiert werden.

Anstelle eines Flüssigkeitsenzugs ist auch eine kontrollierte Flüssigkeitszufuhr in den Patientenkreislauf vorstellbar. Diese kann z. B. durch eine geeignete Ansteuerung der Bilanziereinrichtung 12 durch die Steuereinheit 22 erfolgen. Die Flüssigkeit wird dann über den Dialysator 8 in den Patientenkreislauf gepumpt. Auch eine direkte Flüssigkeitszufuhr in den Patientenkreislauf über eine Pumpe (nicht dargestellt) ist denkbar. Auf diese Weise kann die Bestimmung des Intraperitonealvolumens anstelle über eine Aufkonzentration auch über eine Verdünnung einer körpereigenen Substanz erfolgen.

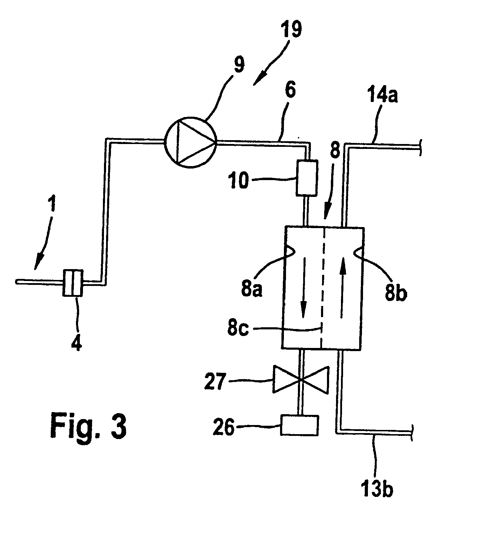

Figur 3 zeigt ein zweites Ausführungsbeispiel der Peritonealdialysevorrichtung. Es sind nur die Teile der Peritonealdialysevorrichtung dargestellt, von denen sich die Vorrichtung von Figur 3 von der Vorrichtung von Figur 1 unterscheidet. Ansonsten ist der Aufbau beider Vorrichtungen gleich. Für gleiche Teile werden gleiche Bezugszeichen verwendet. Die Einrichtung zur Bestimmung des Intraperitonealvolumens von Fig. 3 entspricht derjenigen von Fig. 1.

Die Peritonealdialysevorrichtung von Fig. 3 weist keine Ablaufleitung auf, um Peritoneallösung aus der ersten Kammer 8a des Dialysators in den Peritonealraum des Patienten zurückzuführen. Der Auslaß der ersten Kammer 8a des Dialysators 8 ist mittels eines für Luft durchlässigen, aber für Flüssigkeit undurchlässigen Hydrophobfilters 26 verschlossen. Vor dem Hydrophobfilter ist noch ein Ventil 27 angeordnet. Die Pumpe 9 fördert Peritoneallösung aus dem Peritonealraum des Patienten über einen einlumigen Katheter zunächst in die erste Kammer 8a des Dialysators 8, wobei das Ventil 27 geschlossen ist. Anschließend wird die Drehrichtung der Pumpe 9 umgeschaltet und das Ventil 27 geöffnet, so daß die Pumpe 9 die Peritoneallösung aus der ersten Kammer 8a des Dialysators 8 wieder zurück in den Peritonealraum fördert. Dabei wird die erste Kammer 8a des Dialysators 8 über die Hydrophobmembran 26 belüftet.

Die Bestimmung des Intraperitonealvolumens erfolgt analog zu der unter Bezugnahme auf Fig. 1 beschriebenen Vorrichtung über eine Konzentrationsmessung.

**Patentansprüche**

1. Verfahren zur Bestimmung des Intraperitonealvolumens während einer Peritonealdialyse, bei der in einem ersten Kreislauf Peritoneallösung aus dem Peritonealraum an der einen Seite einer semipermeablen Membran vorbeigeführt und Dialysierflüssigkeit in einem zweiten Kreislauf an der anderen Seite der Membran vorbeigeführt wird, wobei die Konzentration einer Substanz bestimmt und aus der Konzentrationsänderung auf das Intraperitonealvolumen geschlossen wird,
   **dadurch gekennzeichnet, daß**
   die Konzentration einer körpereigenen Substanz, die während der Peritonealdialyse über das Peritoneum in den Peritonealraum übertritt, in der Peritoneallösung bestimmt wird.

2. Verfahren nach Anspruch 1, **dadurch gekennzeichnet, daß** in einer ersten Messung die Konzentration $c_0$ der körpereigenen Substanz in der Peritoneallösung zum Zeitpunkt $t_1$ bestimmt, dem ersten Kreislauf ein vorgegebenes Volumen $\Delta V$ an Flüssigkeit entzogen oder zugeführt und in einer zweiten Messung die Konzentration $c_1$ der Substanz zum Zeitpunkt $t_2$ gemessen wird, wobei das Intraperitonealvolumen aus den Konzentrationen $c_0$ und $c_1$ und dem entzogenen bzw. zugeführten Volumen $\Delta V$ berechnet wird.

3. Verfahren nach Anspruch 2, **dadurch gekennzeichnet, daß** aus der Änderung des Intraperitonealvolumens in dem Zeitraum $t_1 - t_2$ die Ultrafiltrationsrate des Patienten $V(t_1)/t_1$ bestimmt und nach der Bestimmung der Patientenultrafiltrationsrate dem ersten Kreislauf Flüssigkeit mit der Patientenultrafiltrationsrate entzogen wird.

4. Verfahren nach Anspruch 3, **dadurch gekennzeichnet, daß** die Änderung des Intraperitonealvolumens zur Bestimmung der Patientenultrafiltrationsrate während der Peritonealdialyse fortlaufend bestimmt wird.

5. Verfahren nach einem der Ansprüche 1 bis 4, **dadurch gekennzeichnet, daß** die körpereigene Substanz Albumin ist.

6. Vorrichtung zur Peritonealdialyse mit einer Dialysierflüssigkeitsquelle (11) und einem Dialysator (8), der durch eine semipermeable Membran (8c) in eine erste und eine zweite Kammer (8a, 8b) unterteilt ist, wobei die erste Kammer Teil eines ersten Kreislaufs (19) für Peritoneallösung und die zweiter Kammer Teil eines zweiten Kreislaufs (20) für Dialysierflüssigkeit ist, und mit einer Einrichtung (12, 18) zum Entziehen oder Hinzufügen von Flüssigkeit aus dem ersten Kreislauf und einer Einrichtung zur Bestimmung des Intraperitonealvolumens,
   **dadurch gekennzeichnet,**
   **daß** die Einrichtung zur Bestimmung des Intraperitonealvolumens aufweist:

   eine Meßeinheit (10) zur Bestimmung der Konzentration einer körpereigenen Substanz, die während der Peritonealdialyse über das Peritoneum in den Peritonealraum übertritt, und
   eine Rechen- und Auswerteinheit (24), die aus einer Änderung der Konzentration der körpereigenen Substanz das Intraperitonealvolumen bestimmt.

7. Vorrichtung nach Anspruch 6, **dadurch gekennzeichnet, daß** die Einrichtung zur Bestimmung des Intraperitonealvolumens eine mit der Meßeinheit (10), der Rechen- und Auswerteinheit (24) und der Einrichtung (12, 18) zum Entziehen bzw. Hinzufügen von Flüssigkeit zusammenwirkende Steuereinheit (22) umfaßt, die derart ausgebildet ist, daß in einer ersten Messung die Konzentration $c_0$ der körpereigenen Substanz in der Peritoneallösung zum Zeitpunkt $t_1$ bestimmt, dem ersten Kreislauf ein vorgegebenes Volumen $\Delta V$ an Flüssigkeit entzogen oder hinzugefügt und in einer zweiten Messung die Konzentration $c_1$ der Substanz zum Zeitpunkt $t_2$ gemessen wird.

8. Vorrichtung nach Anspruch 7, **dadurch gekennzeichnet, daß** die Rechen- und Auswerteinheit (24) derart ausgebildet ist, daß das Intraperitonealvolumen aus den Konzentrationen $c_0$ und $c_1$ und dem entzogenen Volumen $\Delta V$ berechnet wird.

9. Vorrichtung nach einem der Ansprüche 7 oder 8, **dadurch gekennzeichnet, daß** die Recheneinheit (24) ferner derart ausgebildet ist, daß aus der Änderung des Intraperitonealvolumens in dem Zeitraum $t_1$ - $t_2$ die Ultrafiltrationsrate des Patienten V $(t_1)/t_1$ bestimmt wird, wobei die Steuereinheit ferner derart ausgebildet ist, daß nach der Bestimmung der Patientenultrafiltrationsrate dem ersten Kreislauf (19) Flüssigkeit mit der Patientenultrafiltrationsrate entzogen oder hinzugefügt wird.

10. Vorrichtung nach einem der Ansprüche 6 bis 9, **dadurch gekennzeichnet, daß** die körpereigene Substanz Albumin ist.

## Claims

1. A method for determining the intraperitoneal volume during peritoneal dialysis, wherein in a first circuit peritoneal solution from the peritoneal space is conveyed past the one side of a semipermeable membrane and dialysing fluid in a second circuit is conveyed past the other side of the membrane, whereby the concentration of a substance is determined and the intraperitoneal volume is deduced from the change in concentration,
**characterised in that**
the concentration of an inherent bodily substance, which during the peritoneal dialysis passes via the peritoneum into the peritoneal space, is determined in the peritoneal solution.

2. The method according to claim 1, **characterised in that**, in a first measurement, concentration $c_0$ of the inherent bodily substance in the peritoneal solution is determined at time $t_1$, a preset volume $\Delta V$ of fluid is withdrawn from or fed to the first circuit and, in a second measurement, concentration $c_1$ of the substance is measured at time $t_2$, whereby the intraperitoneal volume is calculated from concentrations $c_0$ and $c_1$ and withdrawn or fed volume $\Delta V$.

3. The method according to claim 2, **characterised in that** the ultrafiltration rate of the patient V $(t_1)/t_1$ is determined from the change in the intraperitoneal volume in the period $t_1$ - $t_2$ and, after the determination of the patient ultrafiltration rate, fluid is withdrawn from the first circuit at the patient ultrafiltration rate.

4. The method according to claim 3, **characterised in that** the change in the intraperitoneal volume is continuously determined in order to determine the patient ultrafiltration rate during the peritoneal dialysis.

5. The method according to any one of claims 1 to 4, **characterised in that** the inherent bodily substance is albumin.

6. A device for peritoneal dialysis with a dialysing fluid source (11) and a dialyser (8), which is divided by a semipermeable membrane (8c) into a first and a second chamber (8a, 8b), whereby the first chamber is part of a first circuit (19) for peritoneal solution and the second chamber is part of a second circuit (20) for dialysing fluid, and with a device (12, 18) for withdrawing or adding fluid from the first circuit and a device for determining the intraperitoneal volume,
**characterised in that**
the device for determining the intraperitoneal volume comprises:

   a measuring unit (10) for determining the concentration of an inherent bodily substance, which during the peritoneal dialysis passes via the peritoneum into the peritoneal space, and
   a computing and evaluation unit (24), which determines the intraperitoneal volume from a change in the concentration of the inherent bodily substance.

7. The device according to claim 6, **characterised in that** the device for determining the intraperitoneal volume comprises a control unit (22) cooperating with the measuring unit (10), the computing and evaluation unit (24) and the device (12, 18) for withdrawing or adding fluid, said control unit being designed in such a way that, in a first measurement, concentration $c_0$ of the inherent bodily substance in the peritoneal solution is determined at time $t_1$, a preset volume $\Delta V$ of fluid is withdrawn from or fed to the first circuit and, in a second measurement, concentration $c_1$ of the substance is measured at time $t_2$.

**8.** The device according to claim 7, **characterised in that** the computing and evaluation unit (24) is designed in such a way that the intraperitoneal volume is calculated from concentrations $c_0$ and $c_1$ and withdrawn volume $\Delta V$.

**9.** The device according to any one of claims 7 or 8, **characterised in that** the computing unit (24) is further designed in such a way that the ultrafiltration rate of the patient V $(t_1)/t_1$ is determined from the change in the intraperitoneal volume in the period $t_1$ - $t_2$, whereby the control unit is further designed in such a way that, after the determination of the patient ultrafiltration rate, fluid is withdrawn from or added to the first circuit (19) at the patient ultrafiltration rate.

**10.** The device according to any one of claims 6 to 9, **characterised in that** the inherent bodily substance is albumin.

**Revendications**

**1.** Procédé de détermination du Volume intrapéritonéal pendant une dialyse péritonéale, où, dans un premier circuit, de la solution péritonéale sortant de l'espace péritonéal est passée sur le premier côté d'une membrane semi-perméable et, dans un deuxième circuit, du liquide de dialyse est passé sur l'autre côté de la membrane, la concentration d'une substance étant déterminée et la variation de concentration permettant de déduire le volume intrapéritonéal, **caractérisé en ce que** la concentration d'une substance corporelle spécifique passant dans l'espace péritonéal en traversant le péritoine pendant la dialyse péritonéale est déterminée dans la solution péritonéale.

**2.** Procédé selon la revendication 1, **caractérisé en ce que**, lors d'une première mesure, la concentration $c_0$ de la substance corporelle spécifique est déterminée dans la solution péritonéale au moment $t_1$, et un volume de liquide $\Delta V$ défini est prélevé du premier circuit ou ajouté à celui-ci, et **en ce que**, lors d'une deuxième mesure, la concentration $c_1$ de la substance est mesurée au moment $t_2$, le volume intrapéritonéal étant calculé à partir des concentrations $c_0$ et $c_1$ et du volume $\Delta V$ prélevé ou ajouté.

**3.** Procédé selon la revendication 2, **caractérisé en ce que** le coefficient d'ultrafiltration du patient $V(t_1)/t_1$ est déterminé à partir de la variation du volume intrapéritonéal pendant la période $t_1$-$t_2$, et **en ce que** du liquide ayant le coefficient d'ultrafiltration du patient est prélevé du premier circuit après détermination du coefficient d'ultrafiltration du patient.

**4.** Procédé selon la revendication 3, **caractérisé en ce que** la variation du volume intrapéritonéal est déterminée en continu pendant la dialyse péritonéale pour la détermination du coefficient d'ultrafiltration du patient.

**5.** Procédé selon l'une des revendications 1 à 4, **caractérisé en ce que** la substance corporelle spécifique est de l'albumine.

**6.** Appareil de dialyse péritonéale avec une source de liquide de dialyse (11) et un dialyseur (8) subdivisé en un premier et un deuxième compartiments (8a, 8b) par une membrane semi-perméable (8c), le premier compartiment faisant partie d'un premier circuit (19) pour la solution péritonéale et le deuxième compartiment faisant partie d'un deuxième circuit (20) pour le liquide de dialyse, et avec un dispositif (12, 18) pour le prélèvement de liquide du premier circuit, ou l'ajout de liquide à celui-ci, et un dispositif pour la détermination du volume intrapéritonéal, **caractérisé en ce que** le dispositif pour la détermination du volume intrapéritonéal comprend :

une unité de mesure (10) pour déterminer la concentration d'une substance corporelle spécifique passant dans l'espace péritonéal en traversant le péritoine pendant la dialyse péritonéale, et
une unité de calcul et d'analyse (24) déterminant le volume intrapéritonéal à partir d'une variation de concentration de la substance corporelle spécifique.

**7.** Appareil selon la revendication 6, **caractérisé en ce que** le dispositif pour la détermination du volume intrapéritonéal comprend une unité de commande (22) coopérant avec l'unité de mesure (10), l'unité de calcul et d'analyse (24) et le dispositif (12, 18) pour le prélèvement de liquide du premier circuit, ou l'ajout de liquide à celui-ci, et qui est réalisée de telle manière que, lors d'une première mesure, la concentration $c_0$ de la substance corporelle spécifique est déterminée dans la solution péritonéale au moment $t_1$, un volume de liquide $\Delta V$ défini est prélevé du premier circuit ou ajouté à celui-ci, et, lors d'une deuxième mesure, la concentration $c_1$ de la substance est mesurée au moment $t_2$.

**8.** Appareil selon la revendication 7, **caractérisé en ce que** l'unité de calcul et d'analyse (24) est réalisée de telle manière que le volume intrapéritonéal est calculé à partir des concentrations $c_0$ et $c_1$ et du volume $\Delta V$ prélevé.

**9.** Appareil selon l'une des revendications 7 ou 8, **caractérisé en ce que** l'unité de calcul (24) est en outre réalisée de telle manière que le coefficient d'ultrafiltration du patient $V(t_1)$-/$t_1$ est déterminé à partir de la variation du volume intrapéritonéal pendant la période $t_1$-$t_2$, l'unité de commande étant en outre réalisée de telle manière que du liquide ayant le coefficient d'ultrafiltration du patient est prélevé du premier circuit (19) ou ajouté à celui-ci après détermination du coefficient d'ultrafiltration du patient.

**10.** Appareil selon l'une des revendications 6 à 9, **caractérisé en ce que** la substance corporelle spécifique est de l'albumine.

Fig. 1

EP 1 197 236 B1

EP 1 197 236 B1

**Albuminkonzentration**

## Fig. 2a

(mit Veränderung der UF-Rate)

(ohne Veränderung der UF-Rate)

$t_1$   $t_2$          $t_3$   $t_4$          Zeit

**Intraperitonealvolumen**

## Fig. 2b

Zeit

**Ultrafiltration (Maschine)**

## Fig. 2c

Zeit

$t_1$  $t_2$          $t_3$  $t_4$

10

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0149001 A
- DE 2838414 A